# EUROPEAN PATENT APPLICATION

(11) **EP 4 159 863 A1**
(43) Date of publication of application: **05.04.2023**
(21) Application number: 21817607.1
(22) Date of filing: 02.06.2021
(51) Int. Cl.: C12N 15/63, C12N 15/67, C12N 15/86, C12N 7/00, C07K 14/075, A61K 35/761, A61K 48/00, A61P 21/00, A61P 25/00

(54) **CODON-OPTIMIZED NUCLEIC ACID ENCODING SMN1 PROTEIN**

(30) Priority: 02.06.2020 RU 2020118148
(71) Applicant: Joint Stock Company "Biocad", Saint Petersburg, 198515 (RU)
(72) Inventor: MADERA, Dmitriy Aleksandrovich, Moscow, 127299 (RU); GERSHOVICH, Pavel Mikhailovich, Saint Petersburg, 198328 (RU); VESELOVA, Anna Sergeevna, Saint Petersburg, 195256 (RU); SHUGAEVA, Tatiana Evgenievna, Moscow, 119021 (RU); LOMUNOVA, Maria Andreevna, Saint Petersburg, 198215 (RU); SHKLIAEVA, Margarita Aleksandrovna, Saint Petersburg, 195276 (RU); MOROZOV, Dmitry Valentinovich, Saint Petersburg, 190000 (RU)
(74) Representative: Held, Stephan
(86) International application number: PCT/RU2021/000238
(87) International publication number: WO 2021/246909

(57) **Abstract**

The present application relates to the fields of genetics, gene therapy, and molecular biology. More specifically, the present invention relates to an isolated codon-optimized nucleic acid that encodes the SMN1 protein (survival motor neuron protein), an expression cassette and a vector based thereon, as well as an AAV9 (adeno-associated virus serotype 9)-based recombinant virus for increasing the expression of the SMN1 gene in target cells, and use thereof.

## Description

### FIELD OF INVENTION

The present application relates to the fields of genetics, gene therapy, and molecular biology. More specifically, the present invention relates to an isolated codon-optimized nucleic acid that encodes the SMN1 protein (survival motor neuron protein), an expression cassette and a vector based thereon, as well as an AAV9 (adeno-associated virus serotype 9)-based recombinant virus for increasing the expression of the SMN1 gene in target cells, and use thereof.

### BACKGROUND OF INVENTION

Spinal muscular atrophy (SMA) is an autosomal recessive neuromuscular disorder caused by mutations in the survival motor neuron 1 (SMN1) gene and loss of encoded SMN protein (Lefebvre et al., Cell (1995) 80:155-165). The lack of SMN results in motor neuron degeneration in the ventral (anterior) horn of the spinal cord, which leads to weakness of the proximal muscles responsible for crawling, walking, neck movement and swallowing, and the involuntary muscles that control breathing and coughing (Sumner C.J., NeuroRx (2006) 3:235-245). Consequently, SMA patients are susceptible to pneumonia and other pulmonary problems such as restrictive lung disease.

Gene therapy is a promising approach to treating spinal muscular atrophy (SMA).

Adeno-associated virus (AAV) vectors are considered effective in CNS gene therapy because they have suitable toxicity and immunogenicity profiles, they may be used in nerve cell transduction, and they are able to mediate long-term expression in the CNS.

Adeno-associated virus (AAV) is a small (20 nm), independent replication-defective, nonenveloped virus. Many different AAV serotypes have been described in human and primates. The adeno-associated virus genome is composed of (+ or -) single-stranded DNA (ssDNA) being about 4,700 nucleotides long. The genomic DNA has terminal inverted repeats (ITRs) at the ends. The genome comprises two open reading frames (ORFs), Rep and Cap comprising several alternative reading frames encoding various protein products. The rep products are essential for AAV replication, whereas three capsid proteins (VP1, VP2, and VP3), along with other alternative products, are encoded by the Cap gene. VP1, VP2, and VP3 are present at 1:1:10 ratio to form an icosahedral capsid (Xie Q. et al. The atomic structure of adeno-associated virus (AAV-2), a vector for human gene therapy. Proc Natl Acad Sci USA, 2002; 99:10405-10410). During recombinant AAV (rAAV) vector production, an expression cassette flanked by ITR is packaged into an AAV capsid. The genes required for AAV replication are not included in the cassette. Recombinant AAV is considered to be one of the safest and most widely used viral vectors for in vivo gene transfer. Vectors can infect cells of multiple tissue types to provide strong and sustained transgene expression. They are also non-pathogenic, and have a low immunogenicity profile (High KA et al., "rAAV human trial experience" Methods Mol Biol. 2011; 807:429-57).

One of the urgent purposes of research in the area of development of effective gene therapy is codon optimization of genes of interest in vectors to achieve the maximum level of expression of the genes of interest, which, in turn, will allow using lower doses of the vector to achieve a significant effect.

One of the properties of the genetic code is degeneracy, i.e. the ability of different codons (trinucleotides) to encode the same amino acid. Such codons that are translated to the same amino acid are called synonymous codons. In natural sequences, one of the synonymous codons is selected randomly in the course of evolution, but the frequencies of usage of synonymous codons are different: each amino acid has more and less preferred ones. Codon optimization is a widely used technique to amplify the production of protein molecules, which provides a rational mapping of one of suitable synonymous codons to each amino acid in a protein sequence. One of the common principles of codon optimization involves the usage of the most frequent codons, whereas other approaches were introduced later, such as harmonization (reproduction of distribution of codon usage frequencies), but they do not always increase productivity. In addition to codon frequencies, the sequence GC content (ratio of guanine and cytosine to the total length of the sequence) may affect the production efficiency, in particular, it was shown that high GC content is associated with increased mRNA levels in mammalian cells Grzegorz Kudla ET AL., High Guanine and Cytosine Content Increases mRNA Levels in Mammalian Cells, June 2006, Volume 4, Issue 6, e180, pp. 933-942). It is further worth noting that stable secondary structure elements of mRNA, i.e. those having a low free folding energy, may reduce the efficiency.

Different variants of codon-optimization of the sequence of a gene of interest may lead to the following (as compared to a wild-type gene):
a) expression levels of the genes of interest will be slightly increased;
b) expression levels of the genes of interest will be significantly increased;
c) expression levels of the genes of interest will remain approximately at the same level;
d) expression levels of the genes of interest will be lowered.

Thus, there is a need for a codon-optimized sequence of the SMN1 gene to increase the expression of the SMN1 gene in target cells.

It was found that the codon-optimized sequence of SMN1 (SMN1-GeneBeam (or abbreviated as SMN1-GB)), which has the nucleotide sequence of SEQ ID NO: 2, surprisingly increases the transcription of the SMN1 gene by more than 3 times, that is, surprisingly increases the mRNA copy number of SMN1-GeneBeam by more than 3 times as compared to SMN1-WT (wild type), which, in turn, leads to a significant increase in the expression of the SMN1 gene and, accordingly, the SMN protein.

### Brief description of invention

In one aspect, the present invention relates to an isolated codon-optimized nucleic acid that encodes the SMN1 protein (survival motor neuron protein) with SEQ ID NO: 1, and includes the nucleic acid sequence of SEQ ID NO: 2.

In one aspect, the present invention relates to an expression cassette that includes the above codon-optimized nucleic acid.

In some embodiments, the expression cassette includes the following elements in the 5'-end to 3'-end direction:
a left (first) ITR (inverted terminal repeats);
a CMV (cytomegalovirus) enhancer;
a CMV (cytomegalovirus) promoter;
an intron of the hBG1 gene (hemoglobin subunit gamma 1 gene)
the above codon-optimized nucleic acid of the SMN1 gene;
an hGH1 polyadenylation signal (human growth hormone gene polyadenylation signal)
a right (second) ITR.

In some embodiments, the expression cassette includes a nucleic acid with the sequence of SEQ ID NO: 4.

In one aspect, the present invention relates to an expression vector that includes the above codon-optimized nucleic acid or the above cassette.

In one aspect, the present invention relates to an AAV9 (adeno-associated virus serotype 9)-based recombinant virus for increasing the expression of the SMN1 gene in target cells, which includes a capsid and the above expression cassette.

In some embodiments, the AAV9-based recombinant virus has a capsid that includes the AAV9 protein VP1.

In some embodiments, the AAV9-based recombinant virus has a capsid that includes the AAV9 protein VP1 having the amino acid sequence of SEQ ID NO: 5.

In some embodiments, the AAV9-based recombinant virus has a capsid that includes the AAV9 protein VP1 having the amino acid sequence of SEQ ID NO: 5 with one or more point mutations.

In some embodiments, the AAV9-based recombinant virus has a capsid that includes the AAV9 protein VP1 having the amino acid sequence of SEQ ID NO: 5 or the amino acid sequence of SEQ ID NO: 5 with one or more point mutations, and the expression cassette includes the following elements in the 5'-end to 3'-end direction:
a CMV enhancer;
a CMV promoter;
an intron of the hBG1 gene;
the above codon-optimized nucleic acid of the SMN1 gene;
an hGH1 polyadenylation signal;
a right ITR.

In some embodiments, the AAV9-based recombinant virus has a capsid that includes the AAV9 protein VP1 having the amino acid sequence of SEQ ID NO: 5 or the amino acid sequence of SEQ ID NO: 5 with one or more point mutations, and the expression cassette comprises a nucleic acid with SEQ ID NO: 4.

In one aspect, the present invention relates to a pharmaceutical composition for delivering the SMN1 gene to target cells, which includes the above AAV9-based recombinant virus in combination with one or more pharmaceutically acceptable excipients.

In one aspect, the present invention relates to the use of the above AAV9-based recombinant virus or the above composition to deliver the SMN1 gene to target cells.

### Brief description of drawings

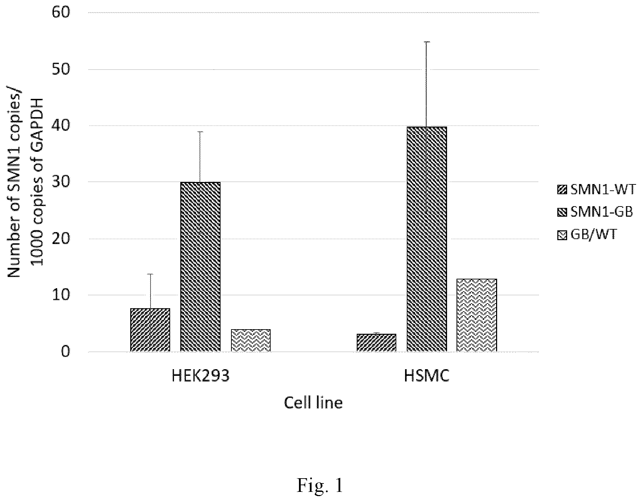

Figure 1. SMN1 expression at the mRNA level following transfection. HEK293 cells and HSMCs were transfected with 5 µg of plasmids pAAV-SMN1-WT and pAAV-SMN1-GB (encoding the SMN1 gene without codon optimization and with codon optimization according to the GeneBeam algorithm). After 72 hours, the copy number of the SMN1 gene in each sample was determined by quantitative PCR (n = 3). The copy number of the GAPDH household gene was also determined. All obtained levels for SMN1 were normalized to 10,000 copies of the GAPDH gene in each sample. Provided is the data on the normalized average copy number of SMN1-WT, SMN-GB for both cell lines, with the indication of a standard deviation. Further provided is the ratio of the normalized copy number of SMN1-GB and SMN1-WT in each line.

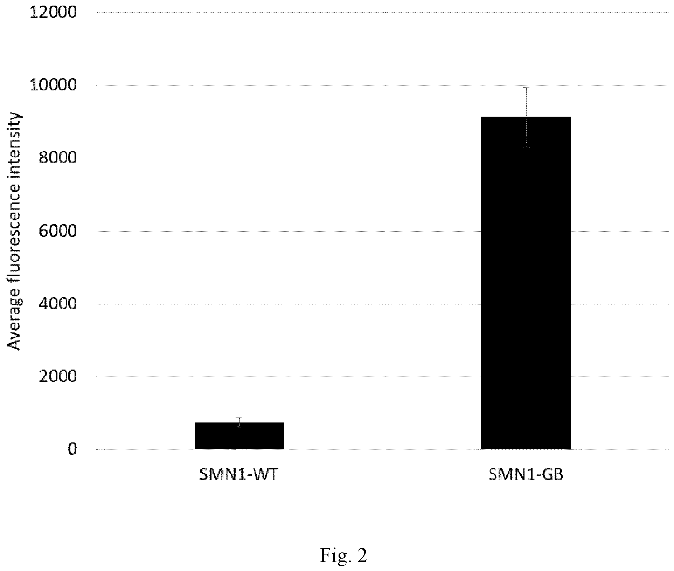

Figure 2. SMN1 expression at the protein level following transfection. HSMCs were transfected with 5 µg of plasmids pAAV-SMN1-WT and pAAV-SMN1-GB (encoding the SMN1 gene without codon optimization and with codon optimization according to the GeneBeam algorithm). After 72 h, the cells were stained with primary antibodies to the SMN1 protein and secondary antibodies labeled with Alexa Fluor 488 in each sample (n = 3). Shown is the average intensity of the fluorescent signal for living cells in the samples after subtracting the background signal obtained on cells stained with secondary antibodies without primary antibodies, with the indication of a standard deviation.

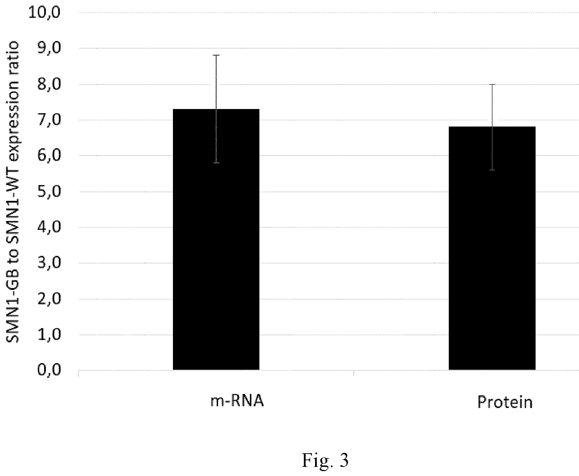

Figure 3. The ratio of SMN1 expression at the mRNA and protein levels following transduction. HSMCs were transduced by AAV9-SMN1-WT and AAV9-SMN1-GB viruses in 3 independent experiments, in each of which the transduction efficiency was at least 50% for the control GFP-containing virus. SMN1 expression was determined at the mRNA and protein levels (see above), following which the ratio of SMN1-GB and SMN1-WT expression was calculated. The figure illustrates average ratios along with standard deviations.

### Definitions and general methods

Unless defined otherwise, all technical and scientific terms used herein have the same meaning as is commonly understood by one of ordinary skill in the art.

Further, unless otherwise required by context, singular terms shall include pluralities and plural terms shall include the singular. Typically, the classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known and widely used by those skilled in the art. Enzyme reactions and purification methods are performed according to the manufacturer's guidelines, as is common in the art, or as described herein.

"Isolated" means altered or removed from the natural state. For example, a nucleic acid or a peptide naturally present in an animal is not "isolated", but the same nucleic acid or peptide partially or completely separated from the coexisting materials of its natural state is "isolated". An isolated nucleic acid or protein can exist in substantially purified form, or can exist in a non-native environment such as, for example, a genetically modified cell.

The terms "naturally occurring," "native," or "wild-type" is used to describe an object that can be found in nature as distinct from being artificially produced. For example, a protein or nucleotide sequence present in an organism (including a virus), which can be isolated from a source in nature and that has not been intentionally modified by a person in the laboratory, is naturally occurring.

The term "genome" refers to the complete genetic material of an organism.

As used in the present description and claims that follow, unless otherwise dictated by the context, the words "include" and "comprise," or variations thereof such as "having," "includes", "including", "comprises," or "comprising," will be understood to imply the inclusion of a stated integer or group of integers but not the exclusion of any other integer or group of integers.

### Protein (Peptide)

As used in the present description, the terms "peptide", "polypeptide" and "protein" are used interchangeably, and they refer to a compound consisting of amino acid residues that are covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used in the present description, the term refers to both short chains, which also commonly are referred to in the art, for example, as peptides, oligopeptides and oligomers, and to longer chains, which generally are referred to in the art as proteins, of which there are many types. "Polypeptides" include, inter alia, for example, biologically active fragments, substantially homologous polypeptides, oligopeptides, homodimers, heterodimers, variants of polypeptides, modified polypeptides, derivatives, analogs, fusion proteins. The polypeptides include natural peptides, recombinant peptides, synthetic peptides, or a combination thereof.

### Nucleic acid molecules

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-stranded DNA or RNA, a single-stranded DNA or RNA, or transcription products of said DNAs.

One skilled in the art has the general knowledge that nucleic acids are polynucleotides that can be hydrolyzed to monomeric "nucleotides". Monomeric nucleotides can be hydrolyzed into nucleosides. As used in the present description, polynucleotides include, as non-limiting examples, all nucleic acid sequences which are obtained by any means available in the art, including, as non-limiting examples, recombinant means, i.e. the cloning of nucleic acid sequences from a recombinant library or a cell genome, using ordinary cloning technology and PCR and the like, and by synthetic means.

It should also be noted here that the present invention does not relate to nucleotide sequences in their natural chromosomal environment, i.e. in a natural state. The sequences of the present invention have been isolated and/or purified, i.e. they were sampled directly or indirectly, for example by a copy, their environment having been at least partially modified. Thus, isolated nucleic acids obtained by recombinant genetics, by means, for example, of host cells, or obtained by chemical synthesis should also be mentioned here.

An "isolated" nucleic acid molecule is one which is identified and separated from at least one nucleic acid molecule-impurity, which the former is typically bound to in the natural source of nuclease nucleic acid. An isolated nucleic acid molecule is different from the form or set in which it is found under natural conditions. Thus, an isolated nucleic acid molecule is different from a nucleic acid molecule that exists in cells under natural conditions. An isolated nucleic acid molecule however includes a nucleic acid molecule located in cells in which the nuclease is normally expressed, for example, if the nucleic acid molecule has a chromosomal localization that is different from its localization in cells under natural conditions.

Unless otherwise indicated, the term nucleotide sequence encompasses its complement. Thus, a nucleic acid having a particular sequence should be understood as one which encompasses the complementary strand thereof with the complementary sequence thereof.

The terms "transformation," "transfection," and "transduction" refer to any method or means by which a nucleic acid is introduced into a cell or host organism, and may be used interchangeably to convey the same meaning. Such methods include, but are not limited to, transfection, electroporation, microinjection, infection, PEG-fusion, and the like.

### Adeno-associated virus (AAV)

Viruses of the Parvoviridae family are small DNA-containing animal viruses. The Parvoviridae family may be divided into two subfamilies: the Parvovirinae, which members infect vertebrates, and the Densovirinae, which members infect insects. By 2006, there have been 11 serotypes of adeno-associated virus described (Mori, S. ET AL., 2004, «Two novel adeno-associated viruses from cynomolgus monkey: pseudotyping characterization of capsid protein», Virology, T. 330 (2): 375-83). All of the known serotypes can infect cells from multiple tissue types. Tissue specificity is determined by the capsid protein serotype; therefore, the adeno-associated virus-based vectors are constructed by assigning the desired serotype. Further information on parvoviruses and other members of the Parvoviridae is described in the literature (Kenneth I. Berns, «Parvoviridae: The Viruses and Their Replication», Chapter 69 in Fields Virology (3d Ed. 1996)).

The genomic organization of all known AAV serotypes is very similar. The genome of AAV is a linear, single-stranded DNA molecule that is less than about 5000 nucleotides (nt) in length. Inverted terminal repeats (ITRs) flank the unique coding nucleotide sequences of replication of non-structural proteins (Rep) and structural proteins (Cap). The Cap gene encodes the VP proteins (VP1, VP2, and VP3) which form the capsid. The terminal 145 nucleotides are self-complementary and are organized such that an energetically stable intramolecular duplex forming a T-shaped hairpin may be formed. Such hairpin structures function as an origin for viral DNA replication, serving as primers for the cellular DNA polymerase complex. Following wild-type AAV (wtAAV) infection in mammalian cells, the Rep genes (e.g. Rep78 and Rep52) are expressed using the P5 promoter and the P19 promoter, respectively, and the both Rep proteins have a certain function in the replication of the viral genome. A splicing event in the Rep open reading frame (Rep ORF) results in the expression of actually four Rep proteins (e.g. Rep78, Rep68, Rep52, and Rep40). However, it has been shown that the unspliced mRNA encoding Rep78 and Rep52 proteins is sufficient for AAV vector production in mammalian cells.

### Vector

The term "vector" as used herein means a nucleic acid molecule capable of transporting another nucleic acid to which it has been linked.

The terms "infection unit (iu)," "infectious particle," or "replication unit," as used in reference to a viral titer, refer to the number of infectious recombinant AAV vector particles as measured by the infectious center assay, also known as replication center assay, as described, for example, in McLaughlin et al., J. Virol. (1988) 62: 1963-1973.

The term "heterologous" as it relates to nucleic acid sequences such as coding sequences and regulatory sequences, denotes sequences that are not normally joined together, and/or are not normally associated with a particular cell. Thus, a "heterologous" region of a nucleic acid construct or a vector is a fragment of nucleic acid within or attached to another nucleic acid molecule that is not found in association with the other molecule in nature. For example, a heterologous region of a nucleic acid construct may include a coding sequence flanked by sequences not found in association with the coding sequence in nature. Another example of a heterologous coding sequence is a construct where the coding sequence itself is not found in nature (e.g. synthetic sequences having codons different from the native gene).

As used in the present description, the term "expression" is defined as the transcription and/or translation of a particular nucleotide sequence driven by its promoter.

### Use

"Gene therapy" is the insertion of genes into subject's cells and/or tissues to treat a disease, typically hereditary diseases, in which a defective mutant allele is replaced with a functional one.

"Treat", "treatment" and "therapy" refer to a method of alleviating or abrogating a biological disorder and/or at least one of attendant symptoms thereof. As used herein, to "alleviate" a disease, disorder or condition means reducing the severity and/or occurrence frequency of the symptoms of a disease, disorder, or condition. Further, references herein to "treatment" include references to curative, palliative and prophylactic treatment.

In one aspect, the subject of treatment, or patient, is a mammal, preferably a human subject. Said subject may be either male or female, of any age.

The term "disorder" means any condition that would benefit from treatment according to the present invention. This includes chronic and acute disorders or diseases including those pathological conditions that predispose the mammal to the disorder in question.

"Disease" is a state of health of an animal where the animal cannot maintain homeostasis, and where if the disease is not ameliorated then the animal's health continues to deteriorate.

The terms "subject," "patient," "individual," and the like are used interchangeably in the present description, and they refer to any animal amenable to the methods described in the present description. In certain non-limiting embodiments, the subject, patient or individual is a human.

"Therapeutically effective amount" refers to that amount of the therapeutic agent being administered during treatment which will relieve to some extent one or more of the symptoms of the disease being treated.

### Detailed description of invention

### Codon-optimized nucleic acid

In one aspect, the present invention relates to an isolated codon-optimized nucleic acid that encodes the SMN1 protein (survival motor neuron protein) with SEQ ID NO: 1, and includes the nucleic acid sequence of SEQ ID NO: 2.

The corresponding amino acid sequence of the SMN_HUMAN protein was used as a basis to produce the codon-optimized SMN1 gene:

MAMSSGGSGGGVPEQEDSVLFRRGTGQSDDSDIWDDTALIKAYDKAVASFKHALKNGDICETS
GKPKTTPKRKPAKKNKSQKKNTAASLQQWKVGDKCSAIWSEDGCIYPATIASIDFKRETCVVVY
TGYGNREEQNLSDLLSPICEVANNIEQNAQENENESQVSTDESENSRSPGNKSDNIKPKSAPWNSF
LPPPPPMPGPRLGPGKPGLKFNGPPPPPPPPPPHLLSCWLPPFPSGPPIIPPPPPICPDSLDDADALGS
MLISWYMSGYHTGYYMGFRQNQKEGRCSHSLN (SEQ ID NO:1)

This amino acid sequence of SEQ ID NO:1 was translated into a nucleotide sequence by sequentially matching each amino acid starting from the N-end of one of the synonymous codons encoding same.

Detailed information on the codon-optimized SMN1 gene and the selection of a final sequence is provided in Example 1.

The final codon-optimized sequence of SMN1 (SMN1-GeneBeam) has the following nucleotide sequence:

ATGGCCATGAGCAGCGGCAGCGGCGGCGTGCCTGAGCAAGAGGACAGCGT
GCTGTTCAGAAGAGGCACCGGCCAGAGCGACGACAGCGACATCTGGGACGACACCGCCCTG
ATCAAGGCCTACGACAAGGCCGTGGCCAGCTTCAAGCACGCCCTGAAGAACGGCGACATCT
GCGAGACCAGCGGCAAGCCCAAGACCACCCCCAAGAGAAAGCCCGCCAAGAAGAACAAGA
GCCAGAAGAAGAACACCGCCGCCAGCCTGCAGCAGTGGAAGGTGGGCGACAAGTGCAGCG
CCATCTGGAGCGAGGACGGCTGCATCTACCCCGCCACCATCGCCAGCATCGACTTCAAGAGA
GAGACCTGCGTGGTGGTGTACACCGGCTACGGCAACAGAGAGGAGCAGAACCTGAGCGACC
TGCTGAGCCCCATCTGCGAGGTGGCCAACAACATCGAGCAGAACGCCCAAGAGAACGAGAA
CGAGAGCCAAGTGAGCACCGACGAGAGCGAGAACAGCAGAAGCCCCGGCAACAAGAGCGA
CAACATCAAGCCCAAGAGCGCCCCCTGGAACAGCTTCCTGCCCCCTCCCCCCCCTATGCCCG
GCCCTAGACTGGGCCCTGGCAAGCCTGGCCTGAAGTTCAACGGCCCCCCCCCCCCTCCTCCT
CCTCCTCCTCCTCACCTGCTGAGCTGCTGGCTGCCCCCCTTCCCCAGCGGCCCTCCTATCATC
CCTCCTCCCCCCCCCATCTGCCCCGACAGCCTGGACGACGCCGACGCCCTGGGCAGCATGCT
GATCAGCTGGTACATGAGCGGCTACCACACCGGCTACTACATGGGCTTCAGACAGAACCAG
AAGGAGGGCCGGTGCAGCCACAGCCTGAACTAG (SEQ ID NO:2).

This final codon-optimized nucleotide sequence of SMN1 (SMN1-GeneBeam) has an increased codon adaptation index (a standard measure for evaluating a sequence for codon frequencies) as compared to the coding sequence of the wild-type SMN gene (SMN1-WT):

ATGGCGATGAGCAGCGGCGGCAGTGGTGGCGGCGTCCCGGAGCAGGAGGATTCCGTGCTGT
TCCGGCGCGGCACAGGCCAGAGCGATGATTCTGACATTTGGGATGATACAGCACTGATAAA
AGCATATGATAAAGCTGTGGCTTCATTTAAGCATGCTCTAAAGAATGGTGACATTTGTGAAA
CTTCGGGTAAACCAAAAACCACACCTAAAAGAAAACCTGCTAAGAAGAATAAAAGCCAAAA
GAAGAATACTGCAGCTTCCTTACAACAGTGGAAAGTTGGGGACAAATGTTCTGCCATTTGGT
CAGAAGACGGTTGCATTTACCCAGCTACCATTGCTTCAATTGATTTTAAGAGAGAAACCTGT
GTTGTGGTTTACACTGGATATGGAAATAGAGAGGAGCAAAATCTGTCCGATCTACTTTCCCC
AATCTGTGAAGTAGCTAATAATATAGAACAAAATGCTCAAGAGAATGAAAATGAAAGCCAA
GTTTCAACAGATGAAAGTGAGAACTCCAGGTCTCCTGGAAATAAATCAGATAACATCAAGC
CCAAATCTGCTCCATGGAACTCTTTTCTCCCTCCACCACCCCCCATGCCAGGGCCAAGACTG
GGACCAGGAAAGCCAGGTCTAAAATTCAATGGCCCACCACCGCCACCGCCACCACCACCAC
CCCACTTACTATCATGCTGGCTGCCTCCATTTCCTTCTGGACCACCAATAATTCCCCCACCAC
CTCCCATATGTCCAGATTCTCTTGATGATGCTGATGCTTTGGGAAGTATGTTAATTTCATGGT
ACATGAGTGGCTATCATACTGGCTATTATATGGGTTTCAGACAAAATCAAAAAGAAGGAAG
GTGCTCACATTCCTTAAATTAA (SEQ ID NO:3).

The codon adaptation index for the final codon-optimized nucleotide sequence of the SMN 1 gene (SEQ ID NO:2) is 98% for the subject sequence, and that is 75% for the wild-type sequence.

The GC content of the wild-type sequence is 45%, i.e. it differs from the target value by 15%, and the GC content of the final codon-optimized nucleotide sequence of the SMN1 gene (SEQ ID NO: 2) for the optimized sequence is 64%, i.e. it differs from the target value by 4%.

The final codon-optimized nucleotide sequence of the SMN1 gene (SEQ ID NO:2) and the nucleotide sequence of the wild-type SMN1 gene (SEQ ID NO:3) are identical by 71%.

### Expression cassette. Expression vector.

In one aspect, the present invention relates to an expression cassette that includes the above codon-optimized nucleic acid.

The term "expression cassette", as used herein, refers in particular to a DNA fragment that is capable, in an appropriate setting, of inducing the expression of a polynucleotide encoding the polypeptide of interest that is included in said expression cassette. When introduced into a host cell, the expression cassette is, inter alia, capable of engaging cellular mechanisms to transcribe the polynucleotide encoding the polypeptide of interest into RNA that is then typically further processed and eventually translated into the polypeptide of interest. The expression cassette may be contained in an expression vector.

The expression cassette of the present invention comprises a promoter as an element. The term "promoter" as used herein refers in particular to a DNA element that promotes the transcription of a polynucleotide to which the promoter is operably linked. The promoter may further form part of a promoter/enhancer element. Although the physical boundaries between the "promoter" and "enhancer" elements are not always clear, the term "promoter" typically refers to a site on the nucleic acid molecule to which an RNA polymerase and/or any associated factors binds and at which transcription is initiated. Enhancers potentiate promoter activity temporally as well as spatially. Many promoters are known in the art to be transcriptionally active in a wide range of cell types. Promoters can be divided into two classes, those that function constitutively and those that are regulated by induction or derepression. The both classes are suitable for protein expression. Promoters that are used for high-level production of polypeptides in eukaryotic cells and, in particular, in mammalian cells, should be strong and preferably active in a wide range of cell types. Strong constitutive promoters which are capable of driving expression in many cell types are well known in the art and, therefore, it is not herein necessary to describe them in detail. In accordance with the idea of the present invention, it is preferable to use the cytomegalovirus (CMV) promoter. A promoter or promoter/enhancer derived from the immediate early (IE) region of human cytomegalovirus (hCMV) is particularly suitable as a promoter in the expression cassette of the present invention. The immediate early (IE) region of human cytomegalovirus (hCMV) and obtained therefrom functional expression-inducing fragments and/or functional expression-augmenting fragments, for example, are described in EP0173177 and EP0323997 and are also well known in the art. Thus, several fragments of the immediate early (IE) region of hCMV may be used as a promoter and/or promoter/enhancer. According to one embodiment of the invention, the human CMV promoter is used in the expression cassette of the present invention.

In some embodiments, the expression cassette includes the following elements in the 5'-end to 3'-end direction:
a left (first) ITR (inverted terminal repeats);
a CMV (cytomegalovirus) enhancer;
a CMV (cytomegalovirus) promoter;
an intron of the hBG1 gene (hemoglobin subunit gamma 1 gene)
the above codon-optimized nucleic acid of the SMN1 gene;
an hGH1 polyadenylation signal (human growth hormone gene polyadenylation signal)
a right (second) ITR.

In some embodiments, the left (first) ITR (inverted terminal repeats) has the following nucleic acid sequence:

Cctgcaggcagctgcgcgctcgctcgctcactgaggccgcccgggcgtcgggcgacctttggtcgcccggcctcagtgagcgagcgagcgcgcag
agagggagtggccaactccatcactaggggttcct (SEQ ID NO: 8).

In some embodiments, the CMV (cytomegalovirus) enhancer has the following nucleic acid sequence:

cgttacataacttacggtaaatggcccgcctggctgaccgcccaacgacccccgcccattgacgtcaataatgacgtatgttcccatagtaacgCcaata
gggactttccattgacgtcaatgggtggagtatttacggtaaactgcccacttggcagtacatcaagtgtatcatatgccaagtacgccccctattgacgtca
atgacggtaaatggcccgcctggcattatgcccagtacatgaccttatgggactttcctacttggcagtacatctacgtattagtcatcgctattaccatg
(SEQ ID NO: 9).

In some embodiments, the CMV (cytomegalovirus) promoter has the following nucleic acid sequence:

gtgatgcggttttggcagtacatcaatgggcgtggatagcggtttgactcacggggatttccaagtctccaccccattgacgtcaatgggagtttgttttgG
caccaaaatcaacgggactttccaaaatgtcgtaacaactccgccccattgacgcaaatgggcggtaggcgtgtacggtgggaggtctatataagcaga
gct (SEQ ID NO: 10).

In some embodiments, the intron of the hBG1 (hemoglobin subunit gamma 1) gene has the following nucleic acid sequence:

cgaatcccggccgggaacggtgcattggaacgcggattccccgtgccaagagtgacgtaagtaccgcctatagagtctataggcccacaaaaaatgctt
tcttcttttaatatacttttttgtttatcttatttctaatactttccctaatctctttctttcagggcaataatgatacaatgtatcatgcctctttgcaccattctaaagaat
aacagtgataatttctgggttaaggcaatagcaatatttctgcatataaatatttctgcatataaattgtaactgatgtaagaggtttcatattgctaatagcagct
acaatccagctaccattctgcttttattttatggttgggataaggctggattattctgagtccaagctaggcccttttgctaatcatgttcatacctcttatcttcctc
ccacagctcctgggcaacgtgctggtctgtgtgctggcccatcactttggcaaagaattgggat (SEQ ID NO: 11).

In some embodiments, the hGH1 (human growth hormone 1 gene) polyadenylation signal has the following nucleic acid sequence:

Acgggtggcatccctgtgacccctccccagtgcctctcctggccctggaagttgccactccagtgcccaccagccttgtcctaataaaattaa
gttgcatcattttgtctgactaggtgtccttctataatattatggggtggaggggggtggtatggagcaaggggcaagttgggaagacaacctgtagggcc
tgcggggtctattgggaaccaagctggagtgcagtggcacaatcttggctcactgcaatctccgcctcctgggttcaagcgattctcctgcctcagcctcc
cgagttgttgggattccaggcatgcatgaccaggctcagctaatttttgtttttttggtagagacggggtttcaccatattggccaggctggtctccaactcct
aatctcaggtgatctacccaccttggcctcccaaattgctgggattacaggcgtgaaccactgctcccttccctgtcctt (SEQ ID NO: 12).

In some embodiments, the right (second) ITR has the following nucleic acid sequence:

aggaacccctagtgatggagttggccactccctctctgcgcgctcgctcgctcactgaggccgggcgaccaaaggtcgcccgacgcccgggctttgcc
cgggcggcctcagtgagcgagcgagcgcgcagctgcctgcagg (SEQ ID NO: 13).

In some embodiments, the expression cassette has the following nucleic acid sequence:

cctgcaggcagctgcgcgctcgctcgctcactgaggccgcccgggcgtcgggcgacctttggtcgcccggcctcagtgagcgagcgagcgcgcaga
gagggagtggccaactccatcactaggggttcctgcggccgcacgcgtctagttattaatagtaatcaattacggggtcattagttcatagcccatatatgg
agttccgcgttacataacttacggtaaatggcccgcctggctgaccgcccaacgacccccgcccattgacgtcaataatgacgtatgttcccatagtaacg
Ccaatagggactttccattgacgtcaatgggtggagtatttacggtaaactgcccacttggcagtacatcaagtgtatcatatgccaagtacgccccctatt
gacgtcaatgacggtaaatggcccgcctggcattatgcccagtacatgaccttatgggactttcctacttggcagtacatctacgtattagtcatcgctattac
catggtgatgcggttttggcagtacatcaatgggcgtggatagcggtttgactcacggggatttccaagtctccaccccattgacgtcaatgggagtttgttt
tgGcaccaaaatcaacgggactttccaaaatgtcgtaacaactccgccccattgacgcaaatgggcggtaggcgtgtacggtgggaggtctatataagc
agagctcgtttagtgaaccgtcagatcgcctggagacgccatccacgctgttttgacctccatagaagacaccgggaccgatccagcctccgcggattc
gaatcccggccgggaacggtgcattggaacgcggattccccgtgccaagagtgacgtaagtaccgcctatagagtctataggcccacaaaaaatgcttt
cttcttttaatatacttttttgtttatcttatttctaatactttccctaatctctttctttcagggcaataatgatacaatgtatcatgcctctttgcaccattctaaagaata
acagtgataatttctgggttaaggcaatagcaatatttctgcatataaatatttctgcatataaattgtaactgatgtaagaggtttcatattgctaatagcagcta
caatccagctaccattctgcttttattttatggttgggataaggctggattattctgagtccaagctaggcccttttgctaatcatgttcatacctcttatcttcctcc
cacagctcctgggcaacgtgctggtctgtgtgctggcccatcactttggcaaagaattgggattcgaacatCGATTGTAATTCATGAGCC
ACCATGGCCATGAGCAGCGGCAGCGGCGGCGTGCCTGAGCAAGAGGACAGCGTGC
TGTTCAGAAGAGGCACCGGCCAGAGCGACGACAGCGACATCTGGGACGACACCGCCCTGAT
CAAGGCCTACGACAAGGCCGTGGCCAGCTTCAAGCACGCCCTGAAGAACGGCGACATCTGC
GAGACCAGCGGCAAGCCCAAGACCACCCCCAAGAGAAAGCCCGCCAAGAAGAACAAGAGC
CAGAAGAAGAACACCGCCGCCAGCCTGCAGCAGTGGAAGGTGGGCGACAAGTGCAGCGCC
ATCTGGAGCGAGGACGGCTGCATCTACCCCGCCACCATCGCCAGCATCGACTTCAAGAGAG
AGACCTGCGTGGTGGTGTACACCGGCTACGGCAACAGAGAGGAGCAGAACCTGAGCGACCT
GCTGAGCCCCATCTGCGAGGTGGCCAACAACATCGAGCAGAACGCCCAAGAGAACGAGAAC
GAGAGCCAAGTGAGCACCGACGAGAGCGAGAACAGCAGAAGCCCCGGCAACAAGAGCGAC
AACATCAAGCCCAAGAGCGCCCCCTGGAACAGCTTCCTGCCCCCTCCCCCCCCTATGCCCGG
CCCTAGACTGGGCCCTGGCAAGCCTGGCCTGAAGTTCAACGGCCCCCCCCCCCCTCCTCCTC
CTCCTCCTCCTCACCTGCTGAGCTGCTGGCTGCCCCCCTTCCCCAGCGGCCCTCCTATCATCC
CTCCTCCCCCCCCCATCTGCCCCGACAGCCTGGACGACGCCGACGCCCTGGGCAGCATGCTG
ATCAGCTGGTACATGAGCGGCTACCACACCGGCTACTACATGGGCTTCAGACAGAACCAGA
AGGAGGGCCGGTGCAGCCACAGCCTGAACTGATctagagtcgacctgcagaagcttgcctcgagcagcgctgctcgag
agatctacgggtggcatccctgtgacccctccccagtgcctctcctggccctggaagttgccactccagtgcccaccagccttgtcctaataaaattaagtt
gcatcattttgtctgactaggtgtccttctataatattatggggtggaggggggtggtatggagcaaggggcaagttgggaagacaacctgtagggcctgc
ggggtctattgggaaccaagctggagtgcagtggcacaatcttggctcactgcaatctccgcctcctgggttcaagcgattctcctgcctcagcctcccga
gttgttgggattccaggcatgcatgaccaggctcagctaatttttgtttttttggtagagacggggtttcaccatattggccaggctggtctccaactcctaatc
tcaggtgatctacccaccttggcctcccaaattgctgggattacaggcgtgaaccactgctcccttccctgtccttctgattttgtaggtaaccacgtgcgga

ccgagcggccgcaggaacccctagtgatggagttggccactccctctctgcgcgctcgctcgctcactgaggccgggcgaccaaaggtcgcccgacg
cccgggctttgcccgggcggcctcagtgagcgagcgagcgcgcagctgcctgcagg (SEQ ID NO: 4).

In one aspect, the present invention relates to an expression vector that includes the above codon-optimized nucleic acid or the above expression cassette.

In some embodiments, the vector is a plasmid, i.e., a circular double stranded piece of DNA into which additional DNA segments may be ligated.

In some embodiments, the vector is a viral vector, wherein additional DNA segments may be ligated into the viral genome.

In some embodiments, vectors are capable of autonomous replication in a host cell into which they are introduced (e.g. bacterial vectors having a bacterial origin site of replication and episomal mammalian vectors). In further embodiments, vectors (e.g. non-episomal mammalian vectors) can be integrated into the genome of a host cell upon introduction into a host cell, and thereby are replicated along with the host gene. Moreover, certain vectors are capable of directing the expression of genes to which they are operably linked. Such vectors are referred to herein as "recombinant expression vectors" (or simply, "expression vectors").

The expression vectors include plasmids, retroviruses, adenoviruses, adeno-associated viruses (AAV), plant viruses, such as cauliflower mosaic virus, tobacco mosaic virus, cosmids, YACs, EBV derived episomes, and the like. DNA molecules may be ligated into the vector such that transcriptional and translational control sequences within the vector serve their intended function of regulating the transcription and translation of the DNA. The expression vector and expression control sequences may be selected to be compatible with the expression host cell used. DNA molecules may be introduced into the expression vector by standard methods (e.g. ligation of complementary restriction sites, or blunt end ligation if no restriction sites are present).

The recombinant expression vector may also encode a signal peptide that facilitates the secretion of the protein of interest from a host cell. The gene of the protein of interest may be cloned into the vector such that the signal peptide is linked in-frame to the amino terminus of the protein of interest. The signal peptide may be an immunoglobulin signal peptide or a heterologous signal peptide (i.e. a signal peptide from a non-immunoglobulin protein).

In addition to the SMN1-GB gene of the present invention, the recombinant expression of the vectors of the present invention may carry regulatory sequences that control the expression of the SMN1-GB gene in a host cell. It will be understood by those skilled in the art that the design of an expression vector, including the selection of regulatory sequences, may depend on such factors as the choice of a host cell to be transformed, the level of expression of a desired protein, and so forth. Preferred control sequences for an expression host cell in mammals include viral elements that ensure high levels of protein expression in mammalian cells, such as promoters and/or enhancers derived from a retroviral LTR, cytomegalovirus (CMV) (such as a CMV promoter/enhancer), simian virus 40 (SV40) (such as a SV40 promoter/enhancer), adenovirus, (e.g. the major late promoter adenovirus (AdMLP)), polyomavirus and strong mammalian promoters such as native immunoglobulin promoter or actin promoter.

The term "control sequences" refers to DNA sequences necessary for the expression of an operably linked coding sequence in a particular host organism. The control sequences that are suitable for prokaryotes, for example, include a promoter, optionally an operator sequence and a ribosome binding site. Eukaryotic cells are known to utilize promoters, polyadenylation signals, and enhancers.

As used in the present description, the term "promoter" or "transcription regulatory sequence" or "regulatory sequence" refers to a nucleic acid fragment that controls the transcription of one or more coding sequences, and that is located upstream with respect to the direction of reading relative to the direction of transcription from the transcription initiation site of the coding sequence, and is structurally identified by the presence of a binding site for DNA-dependent RNA polymerase, transcription initiation sites and any other DNA sequences, including, but not limited to, transcription factor binding sites, repressor and activator protein binding sites, and any other sequences of nucleotides known to one of skill in the art that directly or indirectly regulate the level of transcription with said promoter. A "constitutive" promoter is a promoter that is active in most tissues under typical physiological and developmental conditions. An "inducible" promoter is a promoter that is physiologically or developmentally regulated, e.g. under the influence of a chemical inducer. A "tissue specific" promoter is only active in specific types of tissues or cells.

The terms "enhancers" or "enhancer" as used herein may refer to a DNA sequence that is located adjacent to the DNA sequence that encodes a recombinant product. Enhancer elements are typically located in a 5' direction from a promoter element or can be located downstream of or within a coding DNA sequence (e.g. a DNA sequence transcribed or translated into a recombinant product or products). Hence, an enhancer element can be located 100 base pairs, 200 base pairs, or 300 or more base pairs upstream of a DNA sequence that encodes a recombinant product, or downstream of said sequence. Enhancer elements may increase the amount of a recombinant product being expressed from a DNA sequence above the level of expression associated with a single promoter element. Multiple enhancer elements are readily available to those of ordinary skill in the art.

In addition to the above genes and regulatory sequences, recombinant expression vectors of the invention may carry additional sequences, such as sequences that regulate replication of a vector in host cells (e.g. origins of replication) and selectable marker genes. The selectable marker gene facilitates the selection of host cells into which a vector has been introduced (see e.g. U.S. Patent Nos. 4,399,216, 4,634,665 and 5,179,017). For example, the selectable marker gene typically confers resistance to medicinal agents, such as G418, hygromycin or methotrexate, on a host cell into which the vector has been introduced. For example, selectable marker genes include a dihydrofolate reductase (DHFR) gene (for use in dhfr-host cells during methotrexate selection/amplification), a neo gene (for G418 selection), and a glutamate synthetase gene.

The term "expression control sequence" as used in the present description refers to polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "control sequences" is intended to include at least all components, the presence of which is essential for expression and processing, and can also include additional components, the presence of which is advantageous, for example, leader sequences and fusion partner sequences.

As used herein, the term "operably linked" refers to a linkage of polynucleotide (or polypeptide) elements in a functional relationship. A nucleic acid is "operably linked" when it is present in functional relationship conditions with another nucleic acid sequence. For example, a transcription regulatory sequence is operably linked to a coding sequence if it affects the transcription of said coding sequence. The term "operably linked" means that the DNA sequences being linked are typically contiguous and, where it is necessary to join two protein coding regions, are also contiguous and are present in the reading frame.

In one embodiment of the present invention, "expression vector" relates to a vector comprising one or more polynucleotide sequences of interest, genes of interest, or "transgenes" that are flanked by parvoviral sequences or inverted terminal repeat (ITR) sequences.

Neither the cassette nor the vector of the invention comprises nucleotide sequences of genes encoding non-structural proteins (Rep) and structural proteins (Cap) of the adeno-associated virus.

### AAV9 (adeno-associated virus serotype 9)-based recombinant virus

In one aspect, the present invention relates to an AAV9 (adeno-associated virus serotype 9)-based recombinant virus for increasing the expression of the SMN1 gene in target cells, which includes a capsid and the above expression cassette.

The term "AAV-based recombinant virus" (or "AAV-based virus-like particle", or "AAV recombinant virus strain", or "AAV recombinant vector", or "rAAV vector") as used in this description refers to the above expression cassette (or the above expression vector), which is enclosed within the AAV capsid.

The Cap gene, among other alternative products, encodes 3 capsid proteins (VP1, VP2, and VP3). VP1, VP2, and VP3 are present at 1:1:10 ratio to form an icosahedral capsid (Xie Q. et al. The atomic structure of adeno-associated virus (AAV-2), a vector for human gene therapy. Proc Natl Acad Sci USA, 2002; 99: 10405-10410). Transcription of these genes starts from one promoter, p40. The molecular weights of the corresponding proteins (VP1, VP2 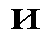 VP3) are 87, 72, and 62 kDa, respectively. All of the three proteins are translated from a single mRNA. Following transcription, pre-mRNA may be spliced in two different manners, where either longer or shorter intron is excised to form mRNAs of various nucleotide lengths.

During the production of the AAV (rAAV)-based recombinant virus, an expression cassette flanked by ITR is packaged into an AAV capsid. The genes required for AAV replication, as mentioned above, are not included in the cassette.

The expression cassette DNA is packaged into a viral capsid in the form of a single stranded DNA molecule (ssDNA) being approximately 3000 nucleotides long. Once a cell is infected with the virus, the single-stranded DNA is converted to the form of double-stranded DNA (dsDNA). The dsDNA can only be used by the cell's proteins, which transcribe the present gene or genes into RNA.

In some embodiments, the AAV9-based recombinant virus has a capsid that includes the AAV9 protein VP1.

In some embodiments, the AAV9-based recombinant virus has a capsid that includes the AAV9 protein VP1 having the following amino acid sequence

MAADGYLPDWLEDNLSEGIREWWALKPGAPQPKANQQHQDNARGLVLPGYKYLGPGNGLDK
GEPVNAADAAALEHDKAYDQQLKAGDNPYLKYNHADAEFQERLKEDTSFGGNLGRAVFQAKK
RLLEPLGLVEEAAKTAPGKKRPVEQSPQEPDSSAGIGKSGAQPAKKRLNFGQTGDTESVPDPQPI
GEPPAAPSGVGSLTMASGGGAPVADNNEGADGVGSSSGNWHCDSQWLGDRVITTSTRTWALPT
YNNHLYKQISNSTSGGSSNDNAYFGYSTPWGYFDFNRFHCHFSPRDWQRLINNNWGFRPKRLNF
KLFNIQVKEVTDNNGVKTIANNLTSTVQVFTDSDYQLPYVLGSAHEGCLPPFPADVFMIPQYGYL
TLNDGSQAVGRSSFYCLEYFPSQMLRTGNNFQFSYEFENVPFHSSYAHSQSLDRLMNPLIDQYLY
YLSKTINGSGQNQQTLKFSVAGPSNMAVQGRNYIPGPSYRQQRVSTTVTQNNNSEFAWPGASSW
ALNGRNSLMNPGPAMASHKEGEDRFFPLSGSLIFGKQGTGRDNVDADKVMITNEEEIKTTNPVA
TESYGQVATNHQSAQAQAQTGWVQNQGILPGMVWQDRDVYLQGPIWAKIPHTDGNFHPSPLM
GGFGMKHPPPQILIKNTPVPADPPTAFNKDKLNSFITQYSTGQVSVEIEWELQKENSKRWNPEIQY
TSNYYKSNNVEFAVNTEGVYSEPRPIGTRYLTRNL (SEQ ID NO: 5).

In some embodiments, the AAV9-based recombinant virus has a capsid that includes the AAV9 protein VP1 having the amino acid sequence of SEQ ID NO: 5 with one or more point mutations.

In some embodiments, the AAV9-based recombinant virus has a capsid that includes the AAV9 protein VP2.

In some embodiments, the AAV9-based recombinant virus has a capsid that includes the AAV9 protein VP2 having the following amino acid sequence:

TAPGKKRPVEQSPQEPDSSAGIGKSGAQPAKKRLNFGQTGDTESVPDPQPIGEPPAAPSGVGSLT
MASGGGAPVADNNEGADGVGSSSGNWHCDSQWLGDRVITTSTRTWALPTYNNHLYKQISNSTS
GGSSNDNAYFGYSTPWGYFDFNRFHCHFSPRDWQRLINNNWGFRPKRLNFKLFNIQVKEVTDN
NGVKTIANNLTSTVQVFTDSDYQLPYVLGSAHEGCLPPFPADVFMIPQYGYLTLNDGSQAVGRS
SFYCLEYFPSQMLRTGNNFQFSYEFENVPFHSSYAHSQSLDRLMNPLIDQYLYYLSKTINGSGQN
QQTLKFSVAGPSNMAVQGRNYIPGPSYRQQRVSTTVTQNNNSEFAWPGASSWALNGRNSLMNP
GPAMASHKEGEDRFFPLSGSLIFGKQGTGRDNVDADKVMITNEEEIKTTNPVATESYGQVATNH
QSAQAQAQTGWVQNQGILPGMVWQDRDVYLQGPIWAKIPHTDGNFHPSPLMGGFGMKHPPPQI

LIKNTPVPADPPTAFNKDKLNSFITQYSTGQVSVEIEWELQKENSKRWNPEIQYTSNYYKSNNVE
FAVNTEGVYSEPRPIGTRYLTRNL (SEQ ID NO: 6).

In some embodiments, the AAV9-based recombinant virus has a capsid that includes the AAV9 protein VP2 having the amino acid sequence of SEQ ID NO: 6 with one or more point mutations.

In some embodiments, the AAV9-based recombinant virus has a capsid that includes the AAV9 protein VP3.

In some embodiments, the AAV9-based recombinant virus has a capsid that includes the AAV9 protein VP3 having the following amino acid sequence

MASGGGAPVADNNEGADGVGSSSGNWHCDSQWLGDRVITTSTRTWALPTYNNHLYKQISNSTS
GGSSNDNAYFGYSTPWGYFDFNRFHCHFSPRDWQRLINNNWGFRPKRLNFKLFNIQVKEVTDN
NGVKTIANNLTSTVQVFTDSDYQLPYVLGSAHEGCLPPFPADVFMIPQYGYLTLNDGSQAVGRS
SFYCLEYFPSQMLRTGNNFQFSYEFENVPFHSSYAHSQSLDRLMNPLIDQYLYYLSKTINGSGQN
QQTLKFSVAGPSNMAVQGRNYIPGPSYRQQRVSTTVTQNNNSEFAWPGASSWALNGRNSLMNP
GPAMASHKEGEDRFFPLSGSLIFGKQGTGRDNVDADKVMITNEEEIKTTNPVATESYGQVATNH
QSAQAQAQTGWVQNQGILPGMVWQDRDVYLQGPIWAKIPHTDGNFHPSPLMGGFGMKHPPPQI
LIKNTPVPADPPTAFNKDKLNSFITQYSTGQVSVEIEWELQKENSKRWNPEIQYTSNYYKSNNVE
FAVNTEGVYSEPRPIGTRYLTRNL (SEQ ID NO: 7).

In some embodiments, the AAV9-based recombinant virus has a capsid that includes the AAV9 protein VP3 having the amino acid sequence of SEQ ID NO: 7 with one or more point mutations.

In some embodiments, the AAV9-based recombinant virus has a capsid that includes the AAV9 proteins VP1, VP2, and VP3.

In some embodiments, the AAV9-based recombinant virus has a capsid that includes the proteins VP1 with the amino acid sequence of SEQ ID NO: 5, VP2 with the amino acid sequence of SEQ ID NO: 6, and VP3 with the amino acid sequence of SEQ ID NO: 7.

In some embodiments, the AAV9-based recombinant virus has a capsid that includes the proteins VP1 with the amino acid sequence of SEQ ID NO: 5 with one or more point mutations, VP2 with the amino acid sequence of SEQ ID NO: 6 with one or more point mutations, and VP3 with the amino acid sequence of SEQ ID NO: 7 with one or more point mutations.

The phrase "more point mutations" refers to two, three, four, five, six, seven, eight, nine, or ten point substitutions.

Particularly preferred embodiments include substitutions (mutations) that are conservative in nature, i.e. substitutions that take place within a family of amino acids that are joined in their side chains. In particular, amino acids are typically divided into four families: (1) acidic amino acids are aspartate and glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. Phenylalanine, tryptophan, and tyrosine are sometimes classified as aromatic amino acids. For example, it is reasonably predictable that an isolated substitution of leucine for isoleucine or valine, an aspartate for a glutamate, a threonine for a serine, or a similar conservative substitution of an amino acid for a structurally related amino acid, will not have a major effect on the biological activity. For example, the polypeptide of interest may include up to about 5-10 conservative or non-conservative amino acid substitutions, so long as the desired function of the molecule remains intact.

An embodiment with point mutations in the sequences of AAV9 proteins VP1, VP2, or VP3 using amino acid substitutions is a substitution of at least one amino acid residue in the AAV9 protein VP1, VP2, or VP3 with another amino acid residue.

Conservative substitutions are shown in Table A under "preferred substitutions".

| Table A | | |
|---|---|---|
| Original residue | Exemplary substitutions | Preferred substitutions |
| Ala (A) | Val; Leu; Ile | Val |
| Arg(R) | Lys; Gin; Asn | Lys |
| Asn(N) | Gin; His; Asp, Lys; Arg | Gin |
| Asp (D) | Glu; Asn | Glu |
| Cys (C) | Ser; Ala | Ser |
| Gln(Q) | Asn; Glu | Asn |
| Glu (E) | Asp; Gin | Asp |
| Gly(G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gin; Asn | Arg |
| Met (M) | Leu; Phe; Ile | Leu |
| Phe(F) | Trp; Leu; Val; Ile; Ala; Tyr | Tyr |
| Pro (P) | Ala | Ala |
| Ser(S) | Thr | Thr |
| Thr (T) | Val; Ser | Ser |
| Trp(W) | Tyr; Phe | Tyr |
| Tyr(Y) | Trp; Phe; Thr; Ser | Phe |
| Val (V) | Ile; Leu; Met; Phe; Ala; Norleucine | Leu |

In some embodiments, the AAV9-based recombinant virus has a capsid that includes the AAV9 protein VP1 having the amino acid sequence of SEQ ID NO: 5 or the amino acid sequence of SEQ ID NO: 5 with one or more point mutations, and the expression cassette includes the following elements in the 5'-end to 3'-end direction:
a CMV enhancer;
a CMV promoter;
an intron of the hBG 1 gene;
the above codon-optimized nucleic acid of the SMN 1 gene;
an hGH1 polyadenylation signal;
a right ITR.

In some embodiments, the AAV9-based recombinant virus has a capsid that includes the proteins VP1 with the amino acid sequence of SEQ ID NO: 5, VP2 with the amino acid sequence of SEQ ID NO: 6, and VP3 with the amino acid sequence of SEQ ID NO: 7, and the expression cassette includes the following elements in the 5'-end to 3'-end direction:
a CMV enhancer;
a CMV promoter;
an intron of the hBG 1 gene;
the above codon-optimized nucleic acid of the SMN 1 gene;
an hGH1 polyadenylation signal;
a right ITR.

In some embodiments, the AAV9-based recombinant virus has a capsid that includes the proteins VP 1 with the amino acid sequence of SEQ ID NO: 5 with one or more point mutations, VP2 with the amino acid sequence of SEQ ID NO: 6 with one or more point mutations, and VP3 with the amino acid sequence of SEQ ID NO: 7 with one or more point mutations, and the expression cassette includes the following elements in the 5'-end to 3'-end direction:
a CMV enhancer;
a CMV promoter;
an intron of the hBG 1 gene;
the above codon-optimized nucleic acid of the SMN 1 gene;
an hGH1 polyadenylation signal;
a right ITR.

In some embodiments, the AAV9-based recombinant virus has a capsid that includes the AAV9 protein VP 1 having the amino acid sequence of SEQ ID NO: 5 or the amino acid sequence of SEQ ID NO: 5 with one or more point mutations, and the expression cassette comprises a nucleic acid with SEQ ID NO: 4.

In some embodiments, the AAV9-based recombinant virus has a capsid that includes the proteins VP1 with the amino acid sequence of SEQ ID NO: 5, VP2 with the amino acid sequence of SEQ ID NO: 6, and VP3 with the amino acid sequence of SEQ ID NO: 7, and the expression cassette comprises a nucleic acid with SEQ ID NO: 4.

In some embodiments, the AAV9-based recombinant virus has a capsid that includes the proteins VP1 with the amino acid sequence of SEQ ID NO: 5 with one or more point mutations, VP2 with the amino acid sequence of SEQ ID NO: 6 with one or more point mutations, and VP3 with the amino acid sequence of SEQ ID NO: 7 with one or more point mutations, and the expression cassette comprises a nucleic acid with SEQ ID NO: 4.

### Pharmaceutical composition

In one aspect, the present invention relates to a pharmaceutical composition for delivering the SMN1 gene to target cells, which includes the above AAV9-based recombinant virus in combination with one or more pharmaceutically acceptable excipients.

In particular embodiments, the present invention relates to a pharmaceutical composition comprising the AAV9-based recombinant virus of the invention in a pharmaceutically acceptable carrier or in other pharmaceutical agents, adjuvants, diluents, etc. For injection, the carrier will typically be a liquid carrier. For other methods of administration, the carrier may be either solid or liquid, such as sterile pyrogen-free water or sterile pyrogen-free phosphate-buffered saline solution. For inhalation administration, the carrier is respirable, and preferably is in a solid or liquid particulate form. As an injection medium, it is preferred to use water that contains the additives that are common for injection solutions, such as stabilizing agents, salts or saline, and/or buffers.

"Pharmaceutical composition" means a composition comprising the above AAV9-based recombinant virus of the invention and at least one of components selected from the group consisting of pharmaceutically acceptable and pharmacologically compatible excipients, such as fillers, solvents, diluents, carriers, auxiliary, distributing agents, delivery agents, preservatives, stabilizers, emulsifiers, suspending agents, thickeners, prolonged delivery controllers, the choice and proportions of which depend on the type and route of administration and dosage. Pharmaceutical compositions of the present invention and methods for preparation thereof will be undoubtedly apparent to those skilled in the art. Pharmaceutical compositions should preferably be manufactured in compliance with the GMP (Good Manufacturing Practice) requirements. A composition may comprise a buffer composition, tonicity agents, stabilizers and solubilizers.

"Pharmaceutically acceptable" means a material that does not have biological or other negative side effects, for example, the material can be administered to a subject without causing any undesirable biological effects. Thus, such pharmaceutical compositions may be used, for example, in transfection of a cell ex vivo or in administration in vivo of the AAV9-based recombinant virus of the invention directly to a subject.

The term "excipient" is used herein to describe any ingredient other than the above ingredients of the invention. These are substances of inorganic or organic nature which are used in the pharmaceutical manufacturing in order to give drug products the necessary physicochemical properties.

"Stabilizer" refers to an excipient or a mixture of two or more excipients that provide the physical and/or chemical stability of the active agent.

The term "buffer", "buffer composition", "buffering agent" refers to a solution, which is capable of resisting changes in pH by the action of its acid-base conjugate components, which allows the rAAV5 vector product to resist changes in pH. Generally, the pharmaceutical composition preferably has a pH in the range from 4,0 to 8.0. Examples of buffers that can be used include, but are not limited to, acetate, phosphate, citrate, histidine, succinate, etc. buffer solutions.

A pharmaceutical composition is "stable" if the active agent retains physical stability and/or chemical stability and/or biological activity thereof during the specified shelf life at storage temperature, for example, of 2-8 °C. Preferably, the active agent retains both physical and chemical stability, as well as biological activity. Storage period is adjusted based on the results of stability test in accelerated or natural aging conditions.

A pharmaceutical composition of the invention can be manufactured, packaged, or widely sold in the form of a single unit dose or a plurality of single unit doses in the form of a ready formulation. The term "single unit dose" as used herein refers to a discrete quantity of a pharmaceutical composition containing a predetermined quantity of an active ingredient. The quantity of the active ingredient typically equals the dose of the active ingredient to be administered in a subject, or a convenient portion of such dose, for example, half or a third of such dose.

### Use

In one aspect, the present invention relates to the use of the above AAV9-based recombinant virus or the above composition to deliver the SMN1 gene to target cells.

Any method for administering the AAV9-based recombinant virus, which is recognized in the art, can be suitably used for the above AAV9-based recombinant virus of the present invention.

The AAV9-based recombinant virus is preferably administered to a cell in a biologically-effective amount. A "biologically-effective" amount of the recombinant virus is an amount that is sufficient to cause infection (or transduction) and expression of the heterologous nucleic acid sequence in the cell. If the virus is administered to a cell in vivo (e.g. the virus is administered to a subject, as described below), a "biologically-effective" amount of the viral vector is an amount that is sufficient to cause the transduction and expression of the heterologous nucleic acid sequence in the target cell.

The cell for administering the above AAV9-based recombinant virus of the invention may be a cell of any type, including but not limited to neural cells (including cells of the peripheral and central nervous systems, in particular, brain cells), lung cells, epithelial cells (e.g. gut and respiratory epithelial cells), muscle cells, pancreatic cells (including islet cells), hepatic cells, myocardial cells, bone cells (e.g. bone marrow stem cells), hematopoietic stem cells, spleen cells, keratinocytes, fibroblasts, endothelial cells, prostate cells, germ cells, and the like. Alternatively, the cell for administering the above AAV9-based recombinant virus may be any progenitor cell. As a further alternative, the cells may be stem cells (e.g. neural stem cells, liver stem cells). Furthermore, the cells may be from any species of origin, as specified above.

The above AAV9-based recombinant virus is not used to modify the genetic integrity of human germ line cells.

### Examples

The following examples are provided for a better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

### Materials and general methods

### Recombinant DNA techniques

DNA manipulations were carried out by standard techniques as described by Sambrook J. et al, Molecular cloning: A laboratory manual; Cold Spring Harbor Laboratory Press, Cold Spring Harbor, New York, 1989. Molecular biological reagents were used according to the manufacturer instructions. Briefly, plasmid DNA was produced for further manipulation in E. coli cells grown under selective antibiotic pressure so that the plasmids were not lost in the cell population. We isolated the plasmid DNA from cells using commercial kits, measured the concentration, and used it for cloning by restriction endonuclease treatment or PCR amplification. The DNA fragments were ligated to each other using ligases and transformed into bacterial cells for the selection of clones and further production. All resulting genetic constructs were confirmed by restriction patterns and complete Sanger sequencing.

### Gene synthesis

Desired gene segments were prepared from oligonucleotides made by chemical synthesis. Gene segments of 300 to 1000 bp long, which were flanked by unique restriction sites, were collected by renaturing oligonucleotides on top of each other, followed by PCR amplification from border primers. As a result, a mixture of fragments was produced, including the desired one. The fragments were cloned at restriction sites into intermediate vectors, following which the DNA sequences of the subcloned fragments were confirmed by DNA sequencing.

### DNA sequence determination

DNA sequences were determined by Sanger sequencing. DNA and protein sequences were analyzed and sequence data was processed in SnapGene Viewer 4.2 or higher for sequence creation, mapping, analysis, annotation and illustration.

### Culturing cell cultures

The experiments used HEK293 (Human Embryonic Kidney clone 293) and HSMC (Human Skeletal Muscle Cells) cell lines. The cells were cultured under standard conditions at 37 C and 5%CO2, on a DMEM complete culture medium supplemented with 10% FBS and an antibiotic. To culture HSMCs, the culture plastic was pre-coated with collagen (Gibco). Cells were subcultured upon reaching 80-90% confluence. Cell viability was assessed using either Trypan Blue stain and a hemocytometer or PI stain and flow cytometry.

### Cell transfection

Cell lines were inoculated the day before transfection into 6-well plates such that they reached 70-80% confluence by the time of transfection. Transfection was performed using commercial lipofection kits according to the manufacturer's protocol. After 72 h, the cells were treated with trypsin solutions or similar, removed from the substrate, washed in a phosphate buffer, and collected for further analysis of expression of target genes and proteins. For each transfection, a control plasmid expressing GFP was used to control the transfection efficiency (percentage of GFP-positive cells). Further analysis was performed only if the transfection efficiency was at least 50%.

All measurements were carried out in 3 independent experiments.

### Gene expression analysis

SMN1 expression at the mRNA level was assessed by quantitative PCR. Briefly, primers and a sample specific for the wild-type SMN1 sequence or GeneBeam were used. Primers and a sample specific for the GAPDH housekeeping gene were used to control the initial RNA levels. Calibration curves were plotted for each set of primers and samples using a known copy number of linearized plasmid DNA comprising the amplified sequence of the corresponding gene. Expression was analyzed by determining, using the calibration curves, the copy number of SMN1-GeneBeam, SMN1-WT, and GAPDH in each sample, following which we normalized the copy number of SMN1 per 10,000 copies of GAPDH. The resulting values were compared for different samples within the same experiment.

### Determination of SMN1 protein expression by flow cytometry

The SMN1 protein content in the cells was assessed by intracellular staining, followed by analysis using flow cytometry. Briefly, the cells were removed from culture plates using TrypLE, washed in PBS, fixed in a 4% paraformaldehyde solution, permeabilized using a 0.5% Triton X-100 solution in PBS, incubated in a blocking buffer supplemented with 1-5% BSA, and stained in two stages using primary antibodies to SMN1 and secondary antibodies labeled with Alexa Fluor 488. Following staining, the cells were washed once in PBS and analyzed on a flow cytometer. The average intensity of the signal was assessed after subtracting the signal stained with secondary antibodies without adding the primary antibodies.

### Assembly and purification of viral particles of recombinant AAV vectors

To assemble AAV particles containing the SMN1 gene or GFP control gene, we used HEK293 packaging cells, into which 3 plasmids were transfected as follows:
A plasmid comprising the AAV genome with a transgene (SNM1 or GFP) expression cassette;
A plasmid for expression of the AAV9 serotype Cap gene and the AAV2 serotype Rep gene. Each gene, using alternative reading frames, encodes several protein products;
A plasmid for expression of Ad5 (adenovirus serotype 5) genes that are required for assembly and packaging of AAV capsids.

After 72 hours, the cells were lysed and the viral particles were purified and concentrated using filtration and chromatography methods. The titer of the viral particles was determined by quantitative PCR with primers and a sample specific for the site of the recombinant viral genome and expressed as the copy number of viral genomes per 1 ml.

### Transducing cell cultures

Cell lines were inoculated similarly to transfection experiments, following which the product with viral particles was added and the cells were analyzed after 72 hours. Transduction efficiency was estimated by measuring the percentage of GFP+ cells.

The cultures being used were pre-tested with the check of the transduction efficiency. Briefly, the AAV9-GFP viral product was transduced into the cell lines in different ratios of cells and viral particles. The ratio of viral particle number to cell number is referred to as multiplicity of infection (MOI). The MOI of the AAV9-GFP virus ranged from 50,000 to 1,000,000. As a result, MOI ranges, within which the transduction efficiency varied linearly depending on MOI, were determined for each line. Further transduction of cell lines was carried out within their linear ranges.

Following transduction, gene and protein expression was analysed as described above.

All measurements were carried out in 3 independent experiments.

### Example 1. Method for producing a codon-optimized SMN1 gene

The corresponding amino acid sequence of the SMN_HUMAN protein (SEQ ID NO: 1) was used as a basis to produce the codon-optimized SMN1 gene.

This amino acid sequence of SEQ ID NO: 1 was translated into a nucleotide sequence by sequentially matching each amino acid starting from the N-end of one of the synonymous codons encoding same with the consideration of one or a combination of the following features:
1) frequency of codon usage (Yasukazu Nakamura ET AL., Codon usage tabulated from the international DNA sequence databases; its status 1999, Nucleic Acids Research, 1999, Vol. 27, No. 1, doi: 10,1093/nar/27,1,292);
2) GC content at the terminal region of the resulting nucleotide sequence (the target value of GC content was 60% as following from the article by Grzegorz Kudla ET AL., High Guanine and Cytosine Content Increases mRNA Levels in Mammalian Cells, PLoS Biol, June 2006, Volume 4, Issue 6, e180, doi: 10.1371/journal.pbio.0040180, so the smaller was the difference between the current GC content and the target one, the more preferable was the codon);
3) free energy of folding of the terminal region of the resulting nucleotide sequence (secondary structures were determined using the Zuker algorithm, Michael Zuker ET AL., Optimal computer folding of large RNA sequences using thermodynamics and auxiliary information, Nucleic Acids Research, Volume 9, Issue 1, 10 January 1981, Pages 133-148, doi: 10.1093/nar/9.1.133).

The construction process further avoided the generation of semantic nucleotide sequences, such as restriction sites, internal ribosome entry sites, and splicing sites.

As a result of translating the amino acid sequence of SEQ ID NO: 1 to a nucleotide sequence, an array of codon-optimized nucleotide sequences of the SMN1 gene was produced.

Several sequences out of the above array of codon-optimized nucleotide sequences of SMN1 did not show an increase in SMN1 gene transcription in further studies, that is, there was no significant increase in the mRNA copy number of SMN1-opt as compared to SMN1-WT on any of the cell lines used, or this increase was insignificant.

Most of the codon-optimized nucleotide sequences of the SMN1 gene showed a 1.5-2-fold increase in the SMN1 gene transcription in further studies, that is, significantly increasing the mRNA copy number of SMN1-opt as compared to SMN1-WT on all the cell lines used.

One sequence out of the above array of codon-optimized nucleotide sequences of the SMN1 gene surprisingly showed more than 3-fold increase in the SMN1 gene transcription in further studies, that is, surprisingly increasing in the mRNA copy number of SMN1-opt by more than 3 times as compared to SMN1-WT on all the cell lines used (see Examples 3-4). This final codon-optimized nucleotide sequence of the SMN1 gene is conventionally called SMN1-GeneBeam (or abbreviated as SMN1-GB).

The final codon-optimized sequence of SMN1 (SMN1-GeneBeam) has a nucleotide sequence represented by SEQ ID NO: 2.

This final codon-optimized nucleotide sequence of SMN1 (SMN1-GeneBeam) is characterized by an increased codon adaptation index (Paul M. Sharp ET AL., The codon adaptation index-a measure of directional synonymous codon usage bias, and its potential applications, Nucleic Acids Research, Volume 15, Issue 3, 11 February 1987, Pages 1281-1295, doi: 10.1093/nar/15.3.1281 - a standard measure for evaluating a sequence for codon usage frequencies) as compared to the coding sequence of the wild-type SMN gene (SMN1-WT with SEQ ID NO: 3).

The codon adaptation index for the final codon-optimized nucleotide sequence of the SMN1 gene (SEQ ID NO: 2) is 98%, and that for the wild-type sequence is 75%.

The GC content of the wild-type sequence is 45%, i.e. it differs from the target value by 15%, and that of the final codon-optimized nucleotide sequence of the SMN1 gene (SEQ ID NO: 2) is 64%, i.e. it differs from the target value by 4%.

The final codon-optimized nucleotide sequence of the SMN1 gene (SEQ ID NO:2) and the nucleotide sequence of the wild-type SMN1 gene (SEQ ID NO:3) are identical by 71%.

### Example 2. Assembly of genetic constructs carrying recombinant AAV genome and encoding SMN1 gene.

A wild-type SMN1 gene sequence was produced by amplification with specific primers with cDNA synthesized based on the total RNA of HEK293 cells. During the amplification process, the Kozak sequence and ClaI restriction site were added from the 5'-end of the gene, and the XbaI restriction site was added from the 3'-end. The sequence of the SMN1 gene was thereafter cloned by the restriction-ligase method at the ClaI and XbaI sites into A commercial construct pAAV-GFP Control plasmid (VPK-402) from CellBiolab (USA), with substitution of the GFP gene with SMN1, thereby producing the pAAV-SMN1-WT construct.

The SMN1-GeneBeam sequence was assembled as described above. In view of sequence complexity, despite its relatively small size, we performed a series of subcloning of gene fragments in intermediate vectors pGEMT, with sequence verification for each vector. Next, a full-length version of the gene was assembled from several intermediate vectors by PCR and cloned into the intermediate vector pGEMT. The construct pAAV-SMN1-WT was used as the final genetic construct, with substitution of the wild-type SMN1 with SMN1-GeneBeam at the ClaI and XbaI sites added to the ends of the SMN1-GeneBeam sequence by PCR.

The final vector contains all the necessary elements for expression and assembly of the gene as part of the recombinant AAV genome:
1) ITRs at the ends of the sequence that is encapsidated into a viral capsid;
2) Elements for expression of the target gene (promoter, enhancer, intron, Kozak sequence, transgene, polyadenylation site);
3) The bacterial replication origin and antibiotic resistance gene to produce plasmid DNA in bacterial cells.

It is important to note that the genetic constructs containing the SMN1-WT and SMN1-GeneBeam genes differ only in the SMN1 gene sequences, and are otherwise completely identical.

### Example 3. Verification of SMN1 expression from genetic constructs.

The genetic constructs pAAV-GFP, pAAV-SMN1-WT, and pAAV-SMN1-GB were transfected into HEK293 cells and HSMCs as described above. We used 5 µg of DNA per 1 well. After 72 h, the cells were collected and the expression of SMN1 (normalized to GAPDH) was analyzed as described above.

It was found that the codon optimization of the SMN1 gene has an effect on the transcription of SMN1, reliably increasing by several times the mRNA copy number of SMN1-GB as compared to that of SMN1-WT on the both cell lines used (Fig. 1). In particular, for HEK293 cells, the normalized expression ratio of SMN1-GB to SMN1-WT was 3.9, whereas, for HSMCs, that was 12.8.

This property of SMN1-GeneBeam, as shown by the data obtained, is not cell-specific, and further provides a several-fold increased expression of the target gene in cells, which can be an important advantage in the development of gene-therapy drugs. Further, this property is not due to any differences in the gene expression cassette and to the properties of appropriate viral capsids carrying the genome from the SMN1-GeneBeam genes, since this analysis was performed on genetic constructs that differ only in the codon optimization of the SMN1 genes, and are otherwise completely identical.

HSMCs were selected to check the expression of SMN1 at the protein level by flow cytometry, as described above. It was shown that the signal from SMN1-specific antibodies in cells transfected with pAAV-SMN1-GB is 12.2 times higher as compared to those transfected with pAAV-SMN1-WT at 5 µg of DNA used per 1 well (Fig. 2). This observation suggests that SMN1-GB has no advantages in translation, but the increased transcription further increases the final protein levels in the cells.

### Example 4. Creatiing viral products expressing SMN1

The plasmids pAAV-SMN1-WT and pAAV-SMN1-GB, along with other plasmids required to produce recombinant AAV viral particles (see above), were used for the bioprocess of AAV production. The serotype used was the wild-type AAV9 serotype or that with one or more point mutations.

In all cases, the properties of the wild-type SMN1 and SMN1-GeneBeam were compared only as long as the serotype used and capsid mutations, if any, were identical. All serotypes based on AAV9, either that of wild type or with mutations, are hereinafter referred to as AAV9 without specifying mutations.

The bioprocess produced recombinant viral particles designated as AAV9-SMN1-WT and AAV9-SMN1-GB, as well as control particles AAV9-GFP. After determining the titers of viral particles, all the 3 products with the same MOI (MOI values varied between experiments from 50,000 to 200,000) were used to transduce permissive cells, i.e. primary human myocytes HSMCs. Further analysis was performed only as long as the transduction efficiency was at least 50%.

Following successful transduction, the cells were removed from the substrate, washed in a phosphate buffer, and the expression of SMN1 was analyzed at the mRNA and protein levels as described above. The increased transcriptional activity of SMN1-GeneBeam was shown to remain consistent; thus, the mRNA of SMN1-GeneBeam was detected to be 7.3 times more than that of wild-type SMN1. A similar increase was further observed at the protein level (6.8 times) (Fig. 3), which shows that there are no advantages of SMN1-GB at the translation level; however, the detectable increase in transcription efficiency provides, using the AAV9-SMN1-GB product, a higher level of SMN1 expression in target cells, which is an important advantage in the treatment of, for example, spinal muscular atrophy, where the level of SMN1 protein expression defines the type of disease from 0 (embryonic lethality) to 4 (no special treatment required).

## Claims

1. A codon-optimized nucleic acid that encodes the SMN1 protein (survival motor neuron protein) with SEQ ID NO: 1, including the nucleic acid sequence of SEQ ID NO: 2.

2. An expression cassette that include the codon-optimized nucleic acid of Claim 1.

3. The expression cassette of Claim 2, including the following elements in the 5'-end to 3'-end direction:
a left (first) ITR (inverted terminal repeats);
a CMV (cytomegalovirus) enhancer;
a CMV (cytomegalovirus) promoter;
an intron of the hBG1 gene (hemoglobin subunit gamma 1 gene)
the codon-optimized nucleic acid of Claim 1;
an hGH1 polyadenylation signal (human growth hormone gene polyadenylation signal)
a right (second) ITR.

4. The expression cassette of Claim 3 that includes a nucleic acid with SEQ ID NO: 4.

5. An expression vector that includes the codon-optimized nucleic acid of Claim 1 or the cassette of Claims 2-4.

6. An AAV9 (adeno-associated virus serotype 9)-based recombinant virus for increasing the expression of the SMN1 gene in target cells, which includes a capsid and the expression cassette of any of Claims 2-4.

7. The AAV9-based recombinant virus of Claim 6, wherein the capsid comprises the AAV9 protein VP1.

8. The AAV9-based recombinant virus of Claim 7, wherein the capsid comprises the AAV9 protein VP1 having the amino acid sequence of SEQ ID NO: 5.

9. The AAV9-based recombinant virus of Claim 7, wherein the capsid comprises the AAV9 protein VP1 having the amino acid sequence of SEQ ID NO: 5 with one or more point mutations.

10. The AAV9-based recombinant virus of Claims 6-9, wherein the capsid comprises the AAV9 protein VP1 having the amino acid sequence of SEQ ID NO: 5 or the amino acid sequence of SEQ ID NO: 5 with one or more point mutations, and the expression cassette includes the following elements in the 5'-end to 3'-end direction:
a CMV enhancer;
a CMV promoter;
an intron of the hBG1 gene;
the codon-optimized nucleic acid of Claim 1;
an hGH1 polyadenylation signal;
a right ITR.

11. The AAV9-based recombinant virus of Claim 6, wherein the capsid includes the AAV9 protein VP1 having the amino acid sequence of SEQ ID NO: 5 or the amino acid sequence of SEQ ID NO: 5 with one or more point mutations, and the expression cassette includes a nucleic acid with SEQ ID NO: 4.

12. A pharmaceutical composition for delivering the SMN1 gene to target cells, including the AAV9-based recombinant virus of Claims 6-11 in combination with one or more pharmaceutically acceptable excipients.

13. Use of the AAV9-based recombinant virus of Claims 6-11 or the composition of Claim 12 for delivering the SMN1 gene to target cells.
